# EUROPEAN PATENT SPECIFICATION

(11) **EP 3 813 954 B1**
(45) Date of publication and mention of the grant of the patent: **26.11.2025**
(21) Application number: 19734389.0
(22) Date of filing: 27.06.2019
(51) Int. Cl.: A61Q 19/00, A61Q 1/00, A61Q 5/00, A61K 8/06, A61K 8/34, A61K 8/44, A61K 8/46, A61K 8/891

(54) **COMPOSITION COMPRISING A SATURATED FATTY ALCOHOL, AT LEAST ONE FATTY-CHAIN ANIONIC SURFACTANT, A POLYOL AND AT LEAST ONE SILICONE OIL**
ZUSAMMENSETZUNG MIT EINEM GESÄTTIGTEN FETTALKOHOL, MINDESTENS EINEM ANIONISCHEN FETTSÄUREKETTENTENSID, EINEM POLYOL UND MINDESTENS EINEM SILIKONÖL
COMPOSITION COMPRENANT UN ALCOOL GRAS SATURÉ, AU MOINS UN TENSIOACTIF ANIONIQUE À CHAÎNE GRASSE, UN POLYOL ET AU MOINS UNE HUILE SILICONÉE

(30) Priority: 28.06.2018 FR 1855860
(43) Date of publication of application: 05.05.2021
(73) Proprietor: L'OREAL, 75008 Paris (FR)
(72) Inventor: SIRICHANDRA, Caroline, 94152 Chevilly La Rue (FR); AUBRUN, Odile, 94152 Chevilly La Rue (FR); BRUNOU, Anne, 94152 Chevilly La Rue (FR); WAHLER, Arno, 94152 Chevilly La Rue (FR)
(74) Representative: Ipsilon
(86) International application number: PCT/EP2019/067229
(87) International publication number: WO 2020/002538

(56) References cited:
- EP-A1- 3 156 037
- US-A1- 2003 228 334
- US-A1- 2010 173 027
- US-A1- 2013 005 835
- US-A1- 2013 189 335

## Description

The present patent application relates to a composition, preferably a cosmetic composition, in the form of an oil-in-water emulsion comprising at least one saturated C16-C18 fatty alcohol; at least one anionic surfactant including a C16-C22 hydrocarbon-based chain; at least one polyol; and at least one silicone oil; in given amounts, and to the use of said composition in the cosmetic and dermatological fields, in particular for caring for or treating keratin materials.

It is known that the skin has a tendency to dry out owing to environmental factors (pollution, wind, cold, air-conditioning), psychological factors (fatigue, stress) or hormonal factors (menopause). However, it is important for the skin to be well hydrated and not to undergo any water loss which risks leading to weathering and drying out of the skin.

Consumers thus expect their cosmetic products to moisturize their skin well.

The feeling of moisturized skin is conveyed, on application, by products that give an aqueous sensation. In the longer term, the feeling of moisturized skin is conveyed by a feeling of supple skin and of moisturized upper epidermal layers. To provide this moisturization of the upper epidermal layers, it is common practice to incorporate humectants, which are hygroscopic substances that bring about rehydration of the skin by uptake of atmospheric water and by retention of water in the skin. Well-known examples of these humectants are the constituents of NMF (Natural Moisturizing Factor), such as urea, or polyols, or else glycols. The higher the content of these humectants, the greater is their moisturizing efficiency.

However, the introduction of these humectants, and more particularly a polyol such as glycerol, into a physiologically acceptable medium brings about impairment of the sensory properties and a reduction in the application quality of the products. Specifically, the introduction of a polyol brings about a reduction, in the aqueous sensation on application. In particular, the compositions are tacky and difficult to apply.

The higher the polyol content, the greater this sensory impairment which causes an impression of a lack of moisturizing efficiency on application.

Moreover, the introduction of these humectants, and more particularly a polyol such as glycerol, into the compositions generally has a negative impact on their stability.

US 2010/173027 and EP 3 156 037 describes emulsion comprising humectant agents. However, these compositions do not solve these above problems.

There is thus still a need to make compositions which have both a moisturizing sensation on application and real moisturizing efficiency on the skin after application (measurable, for example, using a corneometer), and whose stability over time and with respect to temperature is not compromised.

In other words, there is still a need to make cosmetic compositions containing one or more polyols, in particular glycerol, which are both aqueous on application and stable on storage and with respect to temperature variations, including when the polyol concentration is high. The Applicant has discovered that compositions in the form of an oil-in-water emulsion comprising at least one saturated C16-C18 fatty alcohol; at least one anionic surfactant including a C16-C22 hydrocarbon-based chain; at least one polyol; and at least one silicone oil; in given amounts, have not only significant moisturizing efficiency, but also a fresh aqueous sensation on application and good stability.

Thus, one subject of the present invention is a composition, preferably a cosmetic composition, in the form of an oil-in-water emulsion, in which an oily phase is dispersed in an aqueous phase, comprising:
(a) from 2.5% to 7% by weight, relative to the total weight of the composition, of at least one saturated C16-C18 fatty alcohol;
(b) at least one anionic surfactant including a C16-C22 hydrocarbon-based chain;
(c) at least one polyol present in an amount of from 20% and 80% by weight relative to the total weight of the composition;
(d) at least 60% by weight, relative to the total weight of the oily phase, of at least one silicone oil;
the fatty alcohols (a)/anionic surfactants (b) mass ratio being between 10:1 and 4:1.

According to a particular embodiment of the invention, the anionic surfactant(s) contain only one C16-C22 hydrocarbon-based chain. Preferably, the difference between the number of carbon atoms in the hydrocarbon-based chain of the anionic surfactant(s) (b) and the number of carbon atoms in the fatty alcohol(s) (a) must not differ by more than 5 carbon atoms, and is preferentially less than or equal to 4.

The composition according to the invention has the advantage of having a very soft, non-tacky texture which is fresh on application, and is thus very pleasant to use and easy to apply.

Moreover, the composition is stable, and is notably macroscopically and microscopically stable after two months of storage under temperature conditions ranging from 4°C to 45°C, in particular at 4°C, room temperature (generally between 20°C and 25°C), and 45°C.

A subject of the invention is also a process for the cosmetic treatment of keratin materials, which consists in applying to the keratin materials a composition as defined above.

A subject of the invention is also the use of said composition in cosmetics or dermatology, and in particular for caring for, protecting and/or making up bodily or facial skin, or for haircare.

The composition according to the invention is intended for topical application and thus contains a physiologically acceptable medium. The term "physiologically acceptable medium" means here a medium that is compatible with keratin materials.

In the context of the present invention, the term "keratin material" notably means the skin, the scalp, keratin fibres such as the eyelashes, the eyebrows, head hair, bodily hair, the nails, and mucous membranes such as the lips, and more particularly the skin (body, face, area around the eyes, eyelids).

In the text hereinbelow, the expression "at least one" is equivalent to "one or more" and, unless otherwise indicated, the limits of a range of values are included in that range.

### Saturated C16-C18 fatty alcohols

The term "saturated fatty alcohol" means any alcohol comprising a linear saturated (containing no covalent double or triple bonds) hydrocarbon-based chain, in particular a linear alkyl radical, said chain comprising between 16 and 18 carbon atoms and a hydroxyl function.

The term "hydrocarbon-based chain" means an organic group predominantly consisting of hydrogen atoms and carbon atoms.

The saturated fatty alcohol(s) present in the composition in accordance with the invention comprise between 16 and 18 carbon atoms. The saturated fatty alcohol(s) that are of use in the context of the present invention are notably chosen from cetyl alcohol or hexadecanol (C₁₆), stearyl alcohol or octadecanol (C₁₈), which are solid at room temperature and advantageously bear a chain-end -OH group.

Particularly preferably, use will be made of one or more alcohols chosen from cetyl alcohol, stearyl alcohol, and mixtures thereof such as cetylstearyl alcohol.

Preferably, the composition according to the invention comprises a mixture of C₁₆ and C₁₈ fatty alcohols; in particular, the composition comprises cetylstearyl alcohol.

More preferably, the mixture of C₁₆ and C₁₈ fatty alcohols is used in a C₁₆/C₁₈ mass ratio ranging from 20/80 to 80/20 and advantageously in a C₁₆/C₁₈ mass ratio equal to 50/50.

The amount of fatty alcohols in the composition in accordance with the invention is between 2.5% and 7% by weight, preferably between 2.5% and 4.5% by weight and even more preferentially between 2.7% and 4% by weight, relative to the total weight of the composition.

### Anionic surfactants including at least one C16-C22 hydrocarbon-based chain

The anionic surfactant(s) that are useful in the context of the invention comprise at least one saturated (not containing any covalent double or triple bonds) or unsaturated (which may contain one or more covalent double and/or triple bonds), preferably saturated, linear hydrocarbon-based chain, in particular a linear alkyl radical, said chain comprising from 16 to 22 carbon atoms, preferably from 16 to 18 carbon atoms. Preferably, the anionic surfactant(s) that are useful in the context of the invention comprise only one saturated or unsaturated linear hydrocarbon-based chain.

The term "hydrocarbon-based chain" means an organic group predominantly consisting of hydrogen atoms and carbon atoms.

According to a particular embodiment of the invention, the anionic surfactant(s) including at least one C16-C22 hydrocarbon-based chain are chosen from surfactants comprising at least one sulfonate function, acyl glutamates, and mixtures thereof.

According to a preferred embodiment of the invention, the anionic surfactant(s) comprising at least one sulfonate function are chosen from (C16-C22)alkylsulfonates, (C16-C22)alkylamidesulfonates, (C16-C22)alkylarylsulfonates, (C16-C22)alkylsulfoacetates, N-acyl(C16-C22)-N-(C1-C6)alkyltaurates, (C16-C22)acylisethionates, (C16-C22)alkylsulfolaurates, and mixtures thereof.

Preferentially, the anionic surfactant(s) comprising at least one sulfonate function are chosen from:
- acylisethionates, the linear or branched acyl group comprising from 16 to 22 carbon atoms, preferably from 16 to 18 carbon atoms;
- N-acyl-N-alkyltaurates, the linear or branched acyl group comprising from 16 to 22 carbon atoms, preferably from 16 to 18 carbon atoms, and the linear or branched alkyl group comprising from 1 to 6 carbon atoms, or cyclic group comprising from 3 to 6 carbon atoms; preferably, the alkyl group is a methyl,
- and mixtures thereof;
in particular in the form of alkali metal or alkaline-earth metal, ammonium or amino alcohol salts.

As N-acyl-N-alkyltaurates, mention may be made of sodium palmitoyl methyltaurate sold under the name Nikkol PMT^{®} by the company Nikkol; the sodium salt of N-stearoyl N-methyl taurate sold under the name Nikkol SMT by the company Nikko.

According to a preferred embodiment of the invention, the acyl glutamate(s) are chosen from acyl glutamates in which the acyl group comprises from 16 to 18 carbon atoms.

Examples of acyl glutamates that may notably be mentioned include palmitoyl glutamic acid, stearoyl glutamic acid, behenoyl glutamic acid, olivoyl glutamic acid, and the salts of these acids, notably the alkali metal salts such as the Na, Li or K and preferably Na or K salts, the alkaline-earth metal salts such as the Mg salts or the ammonium salts of said acids.

Mention may be made, for example, of the compounds having the INCI name sodium stearoyl glutamate, sodium olivoyl glutamate, and mixtures thereof.

As acylglutamic acid salts, mention may also be made of sodium stearoyl glutamate, such as the product sold under the reference Acylglutamate HS 11 by the company Ajinomoto and disodium hydrogenated stearoyl glutamate, such as the product sold under the reference Acylglutamate HS-21 by the company Ajinomoto.

According to a particular embodiment of the invention, the anionic surfactant(s) including at least one C16-C22 hydrocarbon-based chain are chosen from the surfactants of general formula (I): RCOY(CH₂)ₙSO₃M in which R represents a saturated, linear or branched C16-22 alkyl group; Y represents -O- or -NR₁- with R₁ representing a linear or branched C1-C3 alkyl group; M is chosen from the group formed by hydrogen, alkali metals, alkaline-earth metals, the ammonium group and organic amines; n is an integer ranging from 1 to 3. Preferably, the anionic surfactant of general formula (I) is N-stearoyl-N-methyltaurate, with M corresponding to Na.

The composition according to the invention advantageously comprises from 0.05% to 5%, preferably from 0.1% to 2% and even more preferentially from 0.1% to 1.5% by weight, relative to the total weight of the composition, of one or more anionic surfactants including at least one C16-C22 hydrocarbon-based chain as defined previously.

In the compositions in accordance with the invention, the fatty alcohols (a)/anionic surfactants (b) mass ratio is between 10:1 and 4:1 Preferably, this mass ratio is between 19/2 and 4/1 and even more preferentially between 9/1 and 4/1.

Without wishing to be bound by any theory, it appears that the fatty alcohol(s) and the anionic surfactant(s) including at least one C16-C22 hydrocarbon-based chain form, with the hydrophilic phase, an alpha crystalline phase, also referred to as alpha gel. This phase may notably be characterized by DSC analysis and by X-ray diffraction at a temperature lower than the melting point of the alpha phase gel.

Thus, the composition in accordance with the invention is capable of forming crystalline lamellar phases.

According to a particular embodiment of the invention, the compositions of the invention contain at least one swollen alpha-crystalline phase having a swelling period of greater than 10 nm, preferably greater than 12 nm and even more preferentially greater than or equal to 15 nm. The swelling period is defined as being the sum of the thickness of a bilayer formed by the fatty alcohols (a) and the anionic surfactants (b) and the thickness of the water layer between two leaflets, this unit being repeated several times.

### Polyols

Polyols are defined as being organic molecules comprising at least two hydroxyl (OH) functions.

For the purposes of the invention, the term "polyol" means:
- a saturated or unsaturated, linear or branched hydrocarbon-based chain comprising at least two hydroxyl functions; or
- a saturated, linear or branched hydrocarbon-based chain in which one or more carbon atoms are replaced with an oxygen atom and which comprises at least two hydroxyl functions, for instance polyethylene glycols (PEGs) containing from 4 to 8 ethylene glycol units.

Preferably, the polyol(s) of the composition according to the invention bear a saturated, linear or branched hydrocarbon-based chain.

Advantageously, the polyol(s) comprise a number of carbon atoms ranging from 2 to 20, preferably from 2 to 10, and comprise from 2 to 12 and better still from 2 to 8 hydroxyl functions.

The polyol(s) of the composition according to the invention may be chosen from ethylene glycol, propylene glycol, 1,3-propanediol, isoprene glycol, butylene glycol, dipropylene glycol, polypropylene glycol, glycerol, diglycerol, erythritol, pentaerythritol, arabitol, adonitol, sorbitol, dulcitol, maltitol and panthenol.

Among these polyols, glycerol, propylene glycol, dipropylene glycol, butylene glycol, propane-1,3-diol, and mixtures thereof, are preferentially chosen.

Preferably, the polyol is glycerol.

The polyol(s) are present in the composition in an amount of between 20% and 80% by weight, preferably between 20% and 60% by weight, even more preferentially between 20% and 50% by weight, better still between 20% and 40% by weight, and even better still between 25% and 35% by weight relative to the total weight of the composition.

### Oily phase

The composition according to the invention comprises an oily phase. The proportion of the oily phase of the emulsion may range, for example, from 0.1% to 50% by weight, preferably from 1% to 30% by weight, even more preferentially from 2% to 20% and even better still from 4% to 15%.

This indicated amount does not comprise the content of anionic surfactants as defined previously.

For the purposes of the invention, the oily phase includes any fatty substance that is liquid at room temperature and atmospheric pressure, generally oils, or that is solid at room temperature and atmospheric pressure, like waxes, or any pasty compound, which are present in said composition, with the exception of the fatty alcohols as defined previously.

### Silicone oils

The composition in accordance with the invention comprises at least 60% by weight, relative to the total weight of the oily phase, of at least one silicone oil.

The term "silicone oil" means an oil containing at least one silicon atom, and notably containing Si-O groups.

The silicone oil(s) present in the composition in accordance with the invention may be linear or branched or cyclic. Preferably, they are linear.

The silicone oil(s) present in the composition in accordance with the invention may be volatile or non-volatile.

The term "volatile oil" refers to an oil whose vapour pressure at 25°C and at atmospheric pressure is non-zero, in particular ranging from 0.13 Pa to 40 000 Pa (10⁻³ to 300 mmHg) and preferably ranging from 1.3 Pa to 8000 Pa (0.01 to 60 mmHg).

The term "non-volatile oil" refers to an oil whose vapour pressure at 25°C and at atmospheric pressure is non-zero and is less than 10⁻³ mmHg (0.13 Pa).

Examples of silicone oils that may be mentioned include volatile silicone oils such as cyclopolydimethylsiloxanes (INCI name: cyclomethicone), such as cyclopentasiloxane, cyclohexasiloxane, octamethylcyclotetrasiloxane, decamethylcyclopentasiloxane, dodecamethylcyclohexasiloxane; linear silicones such as heptamethylhexyltrisiloxane, heptamethyloctyltrisiloxane, hexamethyldisiloxane, octamethyltrisiloxane, decamethyltetrasiloxane or dodecamethylpentasiloxane; non-volatile silicone oils such as polymethylsiloxanes (PDMS), and phenyl polymethylsiloxanes such as phenyl trimethicones, phenyl dimethicones, phenyltrimethylsiloxydiphenylsiloxanes, diphenyl dimethicones, diphenylmethyldiphenyltrisiloxanes, 2-phenylethyl trimethyl siloxysilicates and polymethylphenylsiloxanes; polysiloxanes modified with fatty acids, fatty alcohols or polyoxyalkylenes, and mixtures thereof.

According to a particular embodiment of the invention, the composition comprises at least one non-volatile linear silicone oil.

According to a preferred embodiment of the invention, the linear silicone oil(s) are polyorganosiloxanes comprising alkylsiloxane repeating units, the alkyl groups preferably comprising from 1 to 6 carbon atoms and preferably being unsubstituted.

In particular, the silicone oil(s) are chosen from polydimethylsiloxanes (INCI name: dimethicone), preferably of formula: 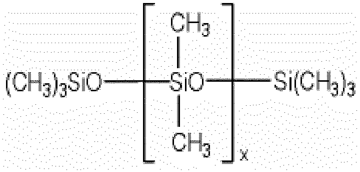 in which x is an integer chosen so as to have a fluid compound.

As linear silicone oils that may be used in the composition according to the invention, examples that may be mentioned include the PDMSs DC 200 Fluid 5 cSt, 10 cSt and 350 cSt sold by the company Dow Corning or the one sold by the company Wacker under the name Wacker Belsil DM 10.

According to a particular embodiment, the silicone oil(s) are present in the composition in accordance with the invention in an amount of between 0.06% and 50% by weight, preferably between 0.6% and 30% by weight, even more preferentially between 1.8% and 20% by weight and better still between 2.4% and 15% by weight relative to the total weight of the composition.

### Additional oils

The composition according to the invention may comprise, besides the silicone oils as described previously, at least one "additional" non-silicone oil.

As non-silicone oils that may be used in the composition of the invention, examples that may be mentioned include:
- hydrocarbon-based oils of animal origin, such as perhydrosqualene (or squalane);
- hydrocarbon-based oils of plant origin, such as liquid triglycerides of fatty acids including from 4 to 10 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, coriander oil, castor oil, avocado oil, caprylic/capric acid triglycerides, for instance those sold by the company Stéarineries Dubois or those sold under the names Miglyol 810, 812 and 818 by the company Dynamit Nobel, jojoba oil, shea butter oil and the liquid fractions of shea butter;
- synthetic esters and ethers, notably of fatty acids or of fatty alcohols, for instance the oils of formulae R¹COOR² and R¹OR² in which R¹ represents a fatty acid residue including from 8 to 29 carbon atoms and R² represents a branched or unbranched hydrocarbon-based chain including from 3 to 30 carbon atoms, for instance purcellin oil, isononyl isononanoate, isopropyl myristate, 2-ethylhexyl palmitate (or octyl palmitate), 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate, triisocetyl citrate, and fatty alcohol heptanoates, octanoates and decanoates; polyol esters, for instance propylene glycol dioctanoate, neopentyl glycol diheptanoate and diethylene glycol diisononanoate; pentaerythritol esters, for instance pentaerythrityl tetraisostearate; and lipophilic amino acid derivatives, such as isopropyl lauroyl sarcosinate (INCI name: Isopropyl Lauroyl sarcosinate) sold under the name Eldew SL 205 by the company Ajinomoto;

- linear or branched hydrocarbons, of mineral or synthetic origin, such as mineral oils (mixture of hydrocarbon-based oils derived from petroleum; INCI name: Mineral oil), volatile or non-volatile liquid paraffins, and derivatives thereof, petroleum jelly, polydecenes, isohexadecane, isododecane, hydrogenated isoparaffin such as hydrogenated polyisobutene for instance Parleam^{®} oil sold by the company NOF Corporation (INCI name: Hydrogenated Polyisobutene);
- fatty alcohols containing from 8 to 26 carbon atoms, for instance cetyl alcohol, stearyl alcohol and mixtures thereof (cetearyl alcohol), octyldodecanol, 2-butyloctanol, 2-hexyldecanol, 2-undecylpentadecanol or oleyl alcohol;
- partially hydrocarbon-based and/or silicone-based fluoro oils, such as those described in JP-A-2-295 912;
- mixtures thereof.

The other fatty substances that may be present in the oily phase are, for example, fatty acids including from 8 to 30 carbon atoms, for instance stearic acid, lauric acid or palmitic acid; gums such as silicone gums (dimethiconol); silicone resins such as trifluoromethyl(C1-4)alkyl dimethicone and trifluoropropyl dimethicone; pastes such as petrolatum; waxes such as microcrystalline waxes, paraffin waxes, lignite waxes, ceresin, ozokerite, montan wax, beeswax, lanolin and derivatives thereof, candelilla wax, ouricury wax, carnauba wax, Japan wax, cocoa butter, palm oil in paste form at 20°C, cork fiber wax, sugar cane wax, hydrogenated oils that are solid at 25°C, fatty esters and glycerides that are solid at 25°C, polyethylene waxes, the waxes obtained by Fischer-Tropsch synthesis, and silicone waxes; and mixtures of these fatty substances.

### Aqueous phase

The composition according to the invention comprises an aqueous phase.

The aqueous phase of the composition in accordance with the invention comprises at least water and the polyol(s) as defined previously. The amount of aqueous phase may range from 50% to 95% by weight, preferably from 60% to 95% by weight and better still from 70% to 90% by weight, relative to the total weight of the composition. The amount of water may represent all or some of the aqueous phase and it is generally at least 25% by weight relative to the total weight of the composition, preferably at least 35% by weight, better still at least 40% by weight.

The water used may be sterile demineralized water and/or a floral water such as rose water, cornflower water, camomile water or lime blossom water, and/or a natural spring water or mineral water, for instance: Vittel water, Vichy basin water, Uriage water, Roche Posay water, Bourboule water, Enghien-les-Bains water, Saint Gervais-les-Bains water, Néris-les-Bains water, Allevar-les-Bains water, Digne water, Maizières water, Neyrac-les-Bains water, Lons-le-Saunier water, Eaux Bonnes water, Rochefort water, Saint Christau water, Fumades water, Tercis-les-bains water and Avène water. The aqueous phase may also comprise reconstituted spring water, i.e. a water containing trace elements such as zinc, copper, magnesium, reconstituting the characteristics of a spring water.

The aqueous (or hydrophilic) phase of the composition according to the invention may also contain any water-soluble or water-dispersible additive.

Water-soluble additives that may also be mentioned include primary alcohols, i.e. an alcohol including from 1 to 6 carbon atoms, such as ethanol and isopropanol. It is preferably ethanol. The addition of such an alcohol may notably be suitable when the composition according to the invention is used as a product for the body or the hair.

The amount of water-soluble or water-dispersible additives in the composition of the invention may range, for example, from 0% to 50% by weight, preferably from 0.5% to 30% by weight and even more preferentially from 2% to 20% by weight, relative to the total weight of the composition.

According to a particular embodiment of the invention, the composition has a pH of between 4 and 9, more preferentially between 4.5 and 7, which makes it possible to optimize its stability and/or its efficacy.

The composition according to the invention is in the form of an oil-in-water emulsion. It includes an oily phase dispersed in an aqueous phase.

In addition, the composition according to the invention may be more or less fluid and may have the appearance of a gel, a white or coloured cream, an ointment, a milk, a lotion, a serum, a paste or a mousse.

In a known manner, all the compositions of the invention may contain one or more of the adjuvants that are common in cosmetics and dermatology: hydrophilic or lipophilic gelling agents and/or thickeners; moisturizers; emollients; hydrophilic or lipophilic active agents; free-radical scavengers; sequestrants; antioxidants; preserving agents; basifying or acidifying agents; fragrances; film-forming agents; dyestuffs (pigments such as iron oxides and titanium dioxide, nacres, and soluble dyes); fillers; and mixtures thereof.

The amounts of these various adjuvants are those conventionally used in the fields under consideration. In particular, the amounts of active agents vary according to the desired aim and are those conventionally used in the fields under consideration, for example from 0.1% to 20% and preferably from 0.5% to 10% by weight relative to the total weight of the composition.

As hydrophilic gelling agents other than the polymers described above, examples that may be mentioned include carboxyvinyl polymers such as the Carbopol products (carbomers) and the Pemulen products (acrylate/C₁₀-C₃₀-alkylacrylate copolymer); polyacrylamides, for instance the crosslinked copolymers sold under the names Sepigel 305 (CTFA name: polyacrylamide/C13-14 isoparaffin/Laureth 7) or Simulgel 600 (CTFA name: acrylamide / sodium acryloyldimethyltaurate copolymer / isohexadecane / polysorbate 80) by the company SEPPIC; cellulose derivatives such as hydroxyethylcellulose; polysaccharides and in particular gums such as xanthan gum and scleroglucan gum; and mixtures thereof. Lipophilic gelling agents that may be mentioned include modified clays such as hectorite and derivatives thereof, for instance the products sold under the name Bentone.

According to a particular embodiment of the invention, the composition is free of hydrophilic gelling polymers. For the purposes of the present invention, the expression "free of hydrophilic gelling polymers" refers to a composition comprising an amount of hydrophilic gelling polymers of between 0 and 1% by weight and preferably between 0 and 0.5% by weight, relative to the total weight of the composition, and even more preferentially between 0 and 0.3%.

### Active agents

The composition of the invention may comprise additional humectants such as sugars, for instance maltose, fructose, sucrose, rhamnose and mannose, hydroxyethylurea such as Hydrovance^{®} sold by the company AkzoNobel, urea, pyrrolidonecarboxylic acid, amino acids and salts thereof, peptides, lactic acid and salts thereof, 4-(2-hydroxyethyl)-1-piperazinemethanesulfonic acid and salts thereof; natural extracts; anti-inflammatory agents; procyannidol oligomers; vitamins such as vitamin A (retinol), vitamin E (tocopherol), vitamin B5 (panthenol), vitamin B3 (niacinamide), derivatives of these vitamins (notably esters) and mixtures thereof; caffeine; depigmenting agents such as kojic acid, hydroquinone and caffeic acid; salicylic acid and derivatives thereof; alpha-hydroxy acids such as lactic acid and glycolic acid and derivatives thereof; retinoids such as carotenoids and vitamin A derivatives; hydrocortisone; melatonin; algal, fungal, plant, yeast or bacterial extracts; steroids; antibacterial active agents; tensioning agents; and mixtures thereof.

The composition of the invention may also comprise oxidation-sensitive hydrophilic active agents. According to the invention, the term "hydrophilic active agent" refers to a compound which has a solubility in water of at least 0.25% at room temperature (25°C). In addition, the term "oxidation-sensitive hydrophilic active agent" refers to any active agent of natural or synthetic origin which is capable of undergoing degradation via an oxidation mechanism. This oxidation phenomenon may have several causes, in particular the presence of oxygen, of light, of metal ions, a high temperature, or certain pH conditions.

Examples of oxidation-sensitive hydrophilic active agents that may be mentioned, in a nonlimiting manner, include ascorbic acid and derivatives thereof such as 5,6-di-O-dimethylsilyl ascorbate (sold by the company Exsymol under the reference PRO-AA), the potassium salt of dl-alpha-tocopheryl-21-ascorbyl phosphate (sold by the company Senju Pharmaceutical under the reference Sepivital EPC), magnesium ascorbyl phosphate, sodium ascorbyl phosphate (sold by the company Roche under the reference Stay-C 50); phloroglucinol; enzymes; and mixtures thereof. According to a preferred embodiment of the invention, use is made, among oxidation-sensitive hydrophilic active agents, of ascorbic acid. The ascorbic acid may be of any nature. Thus, it may be of natural origin in powder form or in the form of orange juice, preferably orange juice concentrate. It may also be of synthetic origin, preferably in powder form.

The composition may also comprise at least one UV-screening agent which may be chosen from hydrophilic, lipophilic or insoluble organic screening agents and/or mineral pigments. It will preferentially be constituted of at least one hydrophilic, lipophilic or insoluble organic UV-screening agent.

As fillers that may be used in the composition of the invention, examples that may be mentioned include pigments such as titanium oxide, zinc oxide or iron oxide and organic pigments; kaolin; silica; talc; boron nitride; organic spherical powders, fibres; and mixtures thereof. Examples of organic spherical powders that may be mentioned include polyamide powders and notably Nylon^{®} powders such as Nylon-1 or Polyamide 12, sold under the name Orgasol by the company Atochem; polyethylene powders; microspheres based on acrylic copolymers, such as those made of ethylene glycol dimethacrylate/lauryl methacrylate copolymer, sold by the company Dow Corning under the name Polytrap; expanded powders such as hollow microspheres and notably the microspheres sold under the name Expancel by the company Kemanord Plast or under the name Micropearl F 80 ED by the company Matsumoto; silicone resin microbeads such as those sold under the name Tospearl by the company Toshiba Silicone; polymethyl methacrylate microspheres, sold under the name Microsphere M-100 by the company Matsumoto or under the name Covabead LH85 by the company Wackherr; ethylene acrylate copolymer powders, such as those sold under the name Flobeads by the company Sumitomo Seika Chemicals; powders of natural organic materials such as starch powders, notably of corn starch, wheat starch or rice starch, which may or may not be crosslinked, such as the starch powders crosslinked with octenyl succinate anhydride, sold under the name Dry-Flo by the company National Starch. Examples of fibres that may be mentioned include polyamide fibres, notably such as Nylon 6 (or Polyamide 6) (INCI name: Nylon 6) fibres, Nylon 6,6 (or Polyamide 66) (INCI name: Nylon 66) fibres, or such as poly-p-phenyleneterephthamide fibres; and mixtures thereof. These fillers may be present in amounts ranging from 0 to 20% by weight and preferably from 0.5% to 10% by weight relative to the total weight of the composition.

Needless to say, a person skilled in the art will take care to select the optional adjuvant(s) added to the composition according to the invention, such that the advantageous properties intrinsically associated with the composition in accordance with the invention are not, or are not substantially, adversely affected by the envisaged addition.

The examples that follow will allow the invention to be understood more clearly, without, however, being limiting in nature. The amounts indicated are weight percentages of starting material, unless otherwise mentioned. The names of the compounds are given as the chemical names.

### EXAMPLES

### Protocol for preparing the compositions

The manufactures are performed with a Triblab or Olsa minilab reactor from the company VMI.

The aqueous phase consisting of the fatty alcohol(s), the anionic surfactant and the preserving agents is introduced into the tank and stirred at 75°C.

The glycerol and the oil are introduced hot at 60°C.

Cooling is performed with stirring under vacuum.

From 35°C, the heat-sensitive starting materials (fragrance, active agent) are added.

The pH is adjusted, if necessary.

The manufacture is stopped at 32°C.

Protocol for evaluating the technical effect of the compositions (stability, performance during/after application, texture, etc.)

### ∘ Protocol for evaluation by a skilled beautician

The application of the compositions and the evaluation of the results are performed by a beautician on six volunteers (Caucasian women):
1) Standardization - Cleansing of standardized skin;
2) Evaluation at T0 of the skin criteria (naked skin) → photo;
3) Standardized application by the beautician (0.30 ml per half-face);
4) Evaluation of the criteria on application;
5) 2 minutes after, evaluation of the result → photos;
6) 1 hour after, evaluation of the result → photos;
7) Evaluation of the finished skin and of the pilling potential.

### o Sensory analysis protocol

The sensory analysis of the compositions is evaluated by touch and visually, by a panel of 17 experts who have normal to combination skin, according to the following protocol. The panel of experts trained in the description of care products evaluates the formulations monadically. The products are packaged in standard 15 mL transparent jars and coded. Within the same session, the samples are presented in random order to each panel member. The application and evaluation of each product are performed on a hand, on a forearm and on a cheek (0.05 ml of product/zone; ten swirls with the index and middle fingers), these zones having been cleansed beforehand.

The glidance of the compositions is evaluated by each expert, who attributes to each composition a score between 0 and 15, and the average of the scores obtained is calculated. The score 0 corresponds to "weak", the score 15 corresponds to "strong".

### ∘ Protocol for measuring the moisturization

The moisturization measurements are taken in vitro on isolated samples of stratum corneum under standardized conditions of relative humidity and of temperature (75% relative humidity and 25°C). The apparatus used is a Corneometer^{®} CM825 corneometer. The machine makes it possible to measure the dielectric capacitance of the stratum corneum (SC), which depends on the mean dielectric permittivity value of the tissue. The dielectric permittivity varies mainly with the amount of water contained in the SC.

The SC samples are conditioned at 75% relative humidity and at 25°C before/during the measurements and the treatment. The emulsions of the invention are deposited on the SC at a rate of 5 µL/cm² before air-drying for a total time of 4 hours. A measurement is taken at T 0h, before the treatment, and a measurement at T = 4h is taken after total drying of the treatment. The variation in the degree of moisturization between T = 0h and T = 4h is calculated. The experiments are performed on four different batches of SC and five technical replicas per condition tested. The results are expressed in arbitrary corneometry units by calculating the mean of all the measurements taken for each test emulsion of the invention.

### o Stability protocol

The "working" characterizations are determined 24 was after manufacture. They concern the macroscopic organoleptic aspects (appearance, colour, odour), the microscopic organoleptic aspects and the physicochemical organoleptic aspects (pH, viscosity).

### Light microscopy

The microscopic observations are performed at room temperature between watch glass and slide using a Leica DMLB microscope which is used in transmission mode. The samples are observed in white light, in polarized light and in differential interferential contrast (DIC) with X20, X40 and X100 lenses.

### The pH

The hydrogen potential is measured after calibration with a Mettler^{®} SevenGo pH-meter (Toledo) with a Mettler - InLabSolids^{®} (Toledo) pH-metric probe at room temperature.

### The viscosity

The viscosity is measured using a Rheomat RM20Lamy viscometer. The measurement calibration is done with a spindle on air. The choice of spindle depends on the viscosity of the product and the measurement must be between 15 and 80 DU (deviation units) in order to be interpretable. Two measurements are taken: one at 30 seconds and one at 10 minutes. The measurement at 10 minutes is indicated in the tables of examples and converted into centipoises.

### The stability monitoring

The stability monitoring is performed on samples after 2 months at 45°C protected from light, and also after 2 months at 4°C protected from light and 2 months at room temperature protected from light. For each stability condition, a macroscopic assessment is performed: appearance (texture, colour, phase separation, appearance of a supernatant, etc.) and odour, along with a viscosity measurement and a microscopic observation (light microscopy in polarized and non-polarized light, magnification of ×400 and ×1000).

### o X-ray diffraction (XRD)

X-ray diffraction (XRD) is a scattering technique which is based on the interactions between X-rays and matter. They are electromagnetic waves whose wavelength ranges between 0.1 Å and 100 Å, which makes it possible to probe matter at the molecular scale. The X-ray diffraction measurements were performed at 20°C, with, for example, a characterization mode using an SAXSpace (Anton Paar) integrating machine:
- a source of X-rays: a tube containing a copper anode is fed by a high-voltage generator set at a voltage of 40 kV and a current of 50 mA.
- an online optical collimating device which optimizes the beam quality (20 × 0.3 mm), confining it in a single direction. The device as a whole (between the source and the detector) is maintained under vacuum.
- a sample holder equipped with a Peltier system for temperature regulation. The sample is conditioned in a PasteCell 1 mm thick.
- a detector consisting of a photosensitive plate.

The exposure time is 2 hours, without obturating the beam (high intensity). The characteristic distances d of the crystalline structures (expressed in Å) are obtained from the relationship *d =* 2*πq* (q = wave vector). The diffraction spectra are analysed in two parts:
□ SAXS (Small Angle X-ray Scattering): measurements at small angles (large distances). The period (Å) characterizing the swelling of the lamellar architecture is measured,
□ WAXS (Wide Angle X-ray Scattering): measurements at large angles (small distances). The intermolecular distance in the lamellar architecture is measured.

### Examples 1 to 7: impact of the content of fatty alcohols and of anionic surfactants

The following compositions are prepared. In these examples, the pH is adjusted to between 4 and 5 by adding sodium hydroxide at the end of manufacture.

| Composition | 1 (invention) | 2 (invention) | 3 (invention) | 4 (comparative) |
|---|---|---|---|---|
| Pure sodium hydroxide | 0.04 | 0.04 | 0.04 | 0.04 |
| Ethylenediaminedisuccinic acid, trisodium salt, as an aqueous 30% solution (Natrlquest E30 from Innospec Active Chemicals) | 0.25 | 0.25 | 0.25 | 0.25 |
| Preserving agent(s) | 0.4 | 0.4 | 0.4 | 0.4 |
| Cetylstearyl alcohol (50/50 C16/C18) (Lanette O OR from BASF) | 6.5 | 4.5 | 2.7 | 1.8 |
| Sodium N-stearoyl-N-methyl taurate (Nikkol SMT from Nikko) | 0.72 | 0.5 | 0.3 | 0.2 |
| Polydimethylsiloxane (viscosity: 5 cSt) (Dowsil SH 200 C Fluid 5 cSt from Dow Corning (Dow Chemical)) | 5 | 5 | 5 | 5 |
| Glycerol | 40 | 40 | 40 | 40 |
| Water | qs 100 | qs 100 | qs 100 | qs 100 |
| Fatty alcohol/surfactant ratio | 9 | 9 | 9 | 9 |

| Composition | 5 (comparative) | 6 (invention) | | 7 (comparative) |
|---|---|---|---|---|
| Pure sodium hydroxide | 0.04 | 0.04 | | 0.04 |
| Ethylenediaminedisuccinic acid, trisodium salt, as an aqueous 30% solution (Natrlquest E30 from Innospec Active Chemicals) | 0.25 | 0.25 | | 0.25 |
| Preserving agent(s) | 0.4 | 0.4 | | 0.4 |
| Cetylstearyl alcohol (50/50 C16/C18) | 1.4 | 2.7 | | 2.7 |
| (Lanette O OR from BASF) | | | | |
| Sodium N-stearoyl-N-methyl taurate | 0.155 | 0.3 | | 0.3 |
| (Nikkol SMT from Nikko) | | | | |
| Polydimethylsiloxane (viscosity: 5 cSt) | 5 | 3 | | - |
| (Dowsil SH 200 C Fluid 5 cSt from Dow Corning (Dow Chemical)) | | | | |
| Squalane of plant origin | - | 2 | | 5 |
| Glycerol | 40 | 40 | | 30 |
| Water | qs 100 | qs 100 | | qs 100 |
| Fatty alcohol/surfactant ratio | 9 | 9 | | 9 |

Compositions 1, 2, 3 and 6 in accordance with the invention show good stability, both microscopically and macroscopically, for two months at 4°C, at room temperature and at 45°C. Comparative compositions 4 and 5, in which the amount of fatty alcohol is not between 2.5% and 7% by weight relative to the total weight of the composition, are unstable. Phase separation is observed macroscopically and the structure is no longer macroscopically homogeneous after two months at 4°C.

Comparative composition 7, the oily phase of which does not contain any silicone oil, is unstable. Phase separation is observed macroscopically and the structure is no longer microscopically homogeneous after two months at 45°C.

### Examples 8 to 11: impact of the glycerol concentration

The following compositions are prepared. In these examples, the pH is adjusted to between 4 and 5 by adding sodium hydroxide at the end of manufacture.

| Composition | 8 (invention) | 9 (invention) | 10 (invention) | **11** (comparative |
|---|---|---|---|---|
| Pure sodium hydroxide | 0.04 | 0.04 | 0.04 | 0.04 |
| Ethylenediaminedisuccinic acid, trisodium salt, as an aqueous 30% solution (Natrlquest E30 from Innospec Active Chemicals) | 0.25 | 0.25 | 0.25 | 0.25 |
| Preserving agent(s) | 0.4 | 0.4 | 0.4 | 0.4 |
| Cetylstearyl alcohol (50/50 C16/C18) | 2.7 | 2.7 | 2.7 | 2.7 |
| (Lanette O OR from BASF) | | | | |
| Sodium N-stearoyl-N-methyl taurate | 0.3 | 0.3 | 0.3 | 0.3 |
| (Nikkol SMT from Nikko) | | | | |
| Polydimethylsiloxane (viscosity: 5 cSt) | 5 | 5 | 5 | 5 |
| (Dowsil SH 200 C Fluid 5 cSt from Dow Corning (Dow Chemical)) | | | | |
| Glycerol | 40 | 30 | 20 | 10 |
| Water | qs 100 | qs 100 | qs 100 | qs 100 |
| Fatty alcohol/surfactant ratio | 9 | 9 | 9 | 9 |

The results obtained are as follows:

| Composition | 8 (invention) | 9 (invention) | 10 (invention) | 11 (comparative) |
|---|---|---|---|---|
| **Sensory analysis** | | | | |
| Glidance | 12/15 | 12/15 | 12/15 | 12/15 |

| **Evaluation by a skilled beautician:** | | | | |
|---|---|---|---|---|
| Glidance | high/very high | high/very high | high/very high | high/very high |
| Dragging effect | very mild/mild | very mild/mild | very mild/mild | very mild/mild |
| Film-forming effect | very mild/mild | very mild/mild | very mild/mild | very mild/mild |
| Pilling | No | No | No | No |
| Moisturization results | 33.9 au | 26.3 au | 29.7 au | 14.3 au |
| Corneometer C825 (arbitrary units) | | | | |

Compositions 8 to 10 in accordance with the invention show good stability, both microscopically and macroscopically, for two months, at 4°C, at room temperature and at 45°C. X-ray diffraction analysis reveals a swelling period of greater than or equal to 15 nm. Compositions 8 to 11 are moreover glidant and easy to apply. They do not show any dragging at the end of application and do not give rise to any pilling. Furthermore, the film-forming effect after application is very mild to mild. Compositions 8 to 10, with a high glycerol content, thus have a sensory nature that is just as advantageous as composition 10 comprising 10% by weight of glycerol.

Furthermore, the moisturization measurement results performed on compositions 8 to 11 show that the formulations are moisturizing and that the moisturization efficiency increases overall as the glycerol content increases. The swollen alpha-gel crystalline lamellar phase structure thus does not impair the moisturizing effect of glycerol.

These results show that the presence of glycerol in the compositions in accordance with the invention, and the increase in the glycerol content, do not have any impact either on the stability of the compositions or on their sensory nature.

### Examples 12 and 13: impact of the fatty alcohols/anionic surfactants ratio

The following compositions are prepared. In these examples, the pH is adjusted to between 4 and 5 by adding sodium hydroxide at the end of manufacture.

| Composition | 12 (comparative) | 13 (invention) |
|---|---|---|
| Pure sodium hydroxide | 0.04 | 0.04 |
| Ethylenediaminedisuccinic acid, trisodium salt, as an aqueous 30% solution (Natrlquest E30 from Innospec Active Chemicals) | 0.25 | 0.25 |
| Preserving agent(s) | 0.4 | 0.4 |
| Cetylstearyl alcohol (50/50 C16/C18) (Lanette O OR from BASF) | 2.4 | 2.7 |
| Sodium N-stearoyl-N-methyl taurate (Nikkol SMT from Nikko) | 0.6 | 0.3 |
| Polydimethylsiloxane (viscosity: 5 cSt) (Dowsil SH 200 C Fluid 5 cSt from Dow Corning (Dow Chemical)) | 5 | 5 |
| Glycerol | 30 | 30 |
| Water | qs 100 | qs 100 |
| Fatty alcohol/surfactant ratio | 4 | 9 |

Composition 13 in accordance with the invention show good stability, both microscopically and macroscopically, for two months, at 4°C, at room temperature and at 45°C. X-ray diffraction analysis reveals a swelling period of greater than or equal to 15 nm.

### Examples 14, 15 and 16: Impact of the nature of the surfactant

The following compositions are prepared.

| Composition | 14 (invention) | 15 (comparative) | 16 (invention) |
|---|---|---|---|
| Pure sodium hydroxide | - | | 0.04 |
| Ethylenediaminedisuccinic acid, trisodium salt, as an aqueous 30% solution | 0.25 | 0.25 | 0.25 |
| (Natrlquest E30 from Innospec Active Chemicals) | | | |
| Preserving agent(s) | 1.1 | 1.1 | 0.4 |
| Cetylstearyl alcohol (50/50 C16/C18) | 2.7 | 2.4 | 2.7 |
| (Lanette O OR from BASF) | | | |
| Sodium N-stearoyl-N-methyl taurate | - | - | 0.3 |
| (Nikkol SMT from Nikko) | | | |
| Monosodium salt of N-stearoyl-L-glutamic acid | 0.3 | 0.6 | - |
| (Acyl glutamate HS 11P from Ajinomoto) | | | |
| Polydimethylsiloxane (viscosity: 5 cSt) | 5 | 5 | 5 |
| (Dowsil SH 200 C Fluid 5 cSt from Dow Corning (Dow Chemical)) | | | |
| Glycerol | 20 | 20 | 20 |
| Water | qs 100 | qs 100 | qs 100 |
| Fatty alcohol/surfactant ratio | 9 | 4 | 9 |

Compositions 14 and 16 in accordance with the invention show good stability, both microscopically and macroscopically, for two months, at 4°C, at room temperature and at 45°C.

### Examples 6 and 17: Impact of the content of the silicone oil

The following compositions are prepared. In these examples, the pH is adjusted to between 4 and 5 by adding sodium hydroxide at the end of manufacture.

| Composition | 6 (invention) | 17 (comparative) |
|---|---|---|
| Pure sodium hydroxide | 0.04 | 0.04 |
| Ethylenediaminedisuccinic acid, trisodium salt, as an aqueous 30% solution (Natrlquest E30 from Innospec Active Chemicals) | 0.25 | 0.25 |
| Preserving agent(s) | 0.4 | 0.4 |
| Cetylstearyl alcohol (50/50 C16/C18) (Lanette O OR from BASF) | 2.7 | 2.7 |
| Sodium N-stearoyl-N-methyl taurate (Nikkol SMT from Nikko) | 0.3 | 0.3 |
| Polydimethylsiloxane (viscosity: 5 cSt) (Dowsil SH 200 C Fluid 5 cSt from Dow Corning (Dow Chemical)) | 3 | 2.5 |
| Squalane of plant origin | 2 | 2.5 |
| Glycerol | 40 | 40 |
| Water | qs 100 | qs 100 |
| Fatty alcohol/surfactant ratio | 9 | 9 |

Composition 6 in accordance with the invention shows good stability, both microscopically and macroscopically, at room temperature and at 45°C.

Comparative composition 17, in which the amount of silicone oil is less than 60% by weight, relative to the total weight of the oily phase, is unstable. Phase separation is observed macroscopically and the structure is no longer macroscopically homogeneous.

## Claims

1. Composition, preferably a cosmetic composition, in the form of an oil-in-water emulsion, in which an oily phase is dispersed in an aqueous phase, comprising:
(a) from 2.5% to 7% by weight, relative to the total weight of the composition, of at least one saturated C16-C18 fatty alcohol;
(b) at least one anionic surfactant including a C16-C22 and preferably C16-C18 hydrocarbon-based chain;
(c) at least one polyol;
(d) at least 60% by weight, relative to the total weight of the oily phase, of at least one silicone oil;
the fatty alcohols (a)/anionic surfactants (b) mass ratio being between 10:1 and 4:1, and in which the polyol(s) are present in an amount of between 20% and 80% by weight, relative to the total weight of the composition.

2. Composition according to Claim 1, in which the anionic surfactant(s) contain only one C16-C22 hydrocarbon-based chain.

3. Composition according to Claim 2, in which the difference between the number of carbon atoms in the hydrocarbon-based chain of the anionic surfactant(s) (b) and the number of carbon atoms in the fatty alcohol(s) (a) must not differ by more than 5 carbon atoms, and is preferentially less than or equal to 4.

4. Composition according to any one of Claims 1 to 3, in which the saturated fatty alcohol(s) are chosen from cetyl alcohol or hexadecanol (C₁₆), stearyl alcohol or octadecanol (C₁₈), which are solid at room temperature and advantageously bear a chain-end -OH group.

5. Composition according to any one of Claims 1 to 4, in which the saturated fatty alcohol(s) are chosen from cetyl alcohol, stearyl alcohol and mixtures thereof such as cetylstearyl alcohol.

6. Composition according to any one of Claims 1 to 5, comprising a mixture of C₁₆ and C₁₈ fatty alcohols, preferably in a C₁₆/C₁₈ mass ratio ranging from 20/80 to 80/20, and advantageously in a C₁₆/C₁₈ mass ratio equal to 50/50.

7. Composition according to any one of Claims 1 to 6, in which the amount of fatty alcohol is between 2.5% and 4.5% by weight and even more preferentially between 2.7% and 4% by weight relative to the total weight of the composition.

8. Composition according to any one of Claims 1 to 7, in which the anionic surfactant(s) including at least one C16-C22 hydrocarbon-based chain are chosen from surfactants comprising at least one sulfonate function, acylglutamates, and mixtures thereof.

9. Composition according to any one of Claims 1 to 8, in which the anionic surfactant(s) including at least one C16-C22 hydrocarbon-based chain are chosen from the surfactants of general formula (I): RCOY(CH₂)ₙSO₃M in which R represents a saturated, linear or branched C16-22 alkyl group; Y represents -O- or -NR₁- with R₁ representing a linear or branched C1-C3 alkyl group; M is chosen from the group formed by hydrogen, alkali metals, alkaline-earth metals, the ammonium group and organic amines; n is an integer ranging from 1 to 3.

10. Composition according to any one of Claims 1 to 9, in which the anionic surfactant including at least one C16-C22 hydrocarbon-based chain is N-stearoyl-N-methyltaurate, with M corresponding to Na.

11. Composition according to any one of Claims 1 to 10, in which the surfactant(s) including at least one C16-C22 hydrocarbon-based chain are present in an amount of between 0.05% and 5% by weight, preferably between 0.1% and 2% by weight and even more preferentially between 0.1% and 1.5% by weight relative to the total weight of the composition.

12. Composition according to any one of Claims 1 to 11, in which the fatty alcohol (a)/anionic surfactants (b) mass ratio is between 19/2 and 4/1 and even more preferentially between 9/1 and 4/1.

13. Composition according to any one of Claims 1 to 12, in which the polyol(s) comprise a number of carbon atoms ranging from 2 to 20 and preferably from 2 to 10.

14. Composition according to any one of Claims 1 to 13, in which the polyol(s) comprise from 2 to 12 hydroxyl functions, preferably from 2 to 8 hydroxyl functions.

15. Composition according to any one of Claims 1 to 14, in which the polyols are chosen from ethylene glycol, propylene glycol, 1,3-propanediol, isoprene glycol, butylene glycol, dipropylene glycol, polypropylene glycol, glycerol, diglycerol, erythritol, pentaerythritol, arabitol, adonitol, sorbitol, dulcitol, maltitol, panthenol, preferably glycerol, propylene glycol, dipropylene glycol, butylene glycol and 1,3-propanediol, and even more preferentially glycerol.

16. Composition according to any one of Claims 1 to 15, in which the polyol(s) are present in an amount of between 20% and 60% by weight, preferentially between 20% and 50% by weight, better still between 20% and 40% by weight, and even better still between 25% and 35% by weight relative to the total weight of the composition.

17. Composition according to any one of Claims 1 to 16, in which the silicone oil(s) are chosen from volatile silicone oils such as cyclopolydimethylsiloxanes (INCI name: cyclomethicone), such as cyclopentasiloxane, cyclohexasiloxane, octamethylcyclotetrasiloxane, decamethylcyclopentasiloxane, dodecamethylcyclohexasiloxane; linear silicones such as heptamethylhexyltrisiloxane, heptamethyloctyltrisiloxane, hexamethyldisiloxane, octamethyltrisiloxane, decamethyltetrasiloxane or dodecamethylpentasiloxane; and non-volatile silicone oils such as polymethylsiloxanes (PDMS), and phenyl polymethylsiloxanes such as phenyl trimethicones, phenyl dimethicones, phenyltrimethylsiloxydiphenylsiloxanes, diphenyl dimethicones, diphenylmethyldiphenyltrisiloxanes, 2-phenylethyl trimethyl siloxysilicates and polymethylphenylsiloxanes; polysiloxanes modified with fatty acids, fatty alcohols or polyoxyalkylenes, and mixtures thereof.

18. Composition according to any one of Claims 1 to 17, comprising at least one non-volatile silicone oil, preferably a silicone oil chosen from polymethylsiloxanes.

19. Composition according to any one of Claims 1 to 18, in which the silicone oil(s) are present in an amount of between 0.06% and 50% by weight, preferably between 0.6% and 30% by weight, even more preferentially between 1.8% and 20% by weight and better still between 2.4% and 15% by weight relative to the total weight of the composition.

20. Cosmetic process for treating a keratin material, in which a composition as defined in any one of Claims 1 to 19 is applied to the keratin material.

21. Use of a composition as defined in any one of Claims 1 to 19, in the cosmetics field, and in particular for caring for, protecting and/or making up bodily or facial skin, or for haircare.

## Patentansprüche

1. Zusammensetzung, vorzugsweise kosmetische Zusammensetzung, in Form einer Öl-in-Wasser-Emulsion, in der eine ölige Phase in einer wässrigen Phase dispergiert ist, umfassend:
(a) 2,5 bis 7 Gew.-%, bezogen auf das Gesamtgewicht der Zusammensetzung, mindestens eines gesättigten C16-C18-Fettalkohols;
(b) mindestens ein anionisches Tensid mit einer kohlenwasserstoffbasierten C16-C22- und vorzugsweise C16-C18-Kette;
(c) mindestens ein Polyol;
(d) mindestens 60 Gew.-%, bezogen auf das Gesamtgewicht der öligen Phase, mindestens eines Silikonöls;
wobei das Massenverhältnis von Fettalkoholen (a) zu anionischen Tensiden (b) zwischen 10:1 und 4:1 liegt und wobei das Polyol bzw. die Polyole in einer Menge zwischen 20 und 80 Gew.-%, bezogen auf das Gesamtgewicht der Zusammensetzung, vorliegt bzw. vorliegen.

2. Zusammensetzung nach Anspruch 1, wobei das anionische Tensid bzw. die anionischen Tenside nur eine kohlenwasserstoffbasierte C16-C22-Kette enthält bzw. enthalten.

3. Zusammensetzung nach Anspruch 2, wobei die Differenz zwischen der Zahl der Kohlenstoffatome in der kohlenwasserstoffbasierten Kette des anionischen Tensids bzw. der anionischen Tenside (b) und der Zahl der Kohlenstoffatome in dem Fettalkohol bzw. den Fettalkoholen (a) um nicht mehr als 5 Kohlenstoffatome verschieden sein darf und vorzugsweise kleiner oder gleich 4 ist.

4. Zusammensetzung nach einem der Ansprüche 1 bis 3, wobei der gesättigte Fettalkohol bzw. die gesättigten Fettalkohole aus Cetylalkohol oder Hexadecanol (C₁₆), Stearylalkohol oder Octadecanol (C₁₈), die bei Raumtemperatur fest sind und vorteilhafterweise eine - OH-Gruppe am Kettenende tragen, ausgewählt ist bzw. sind.

5. Zusammensetzung nach einem der Ansprüche 1 bis 4, wobei der gesättigte Fettalkohol bzw. die gesättigten Fettalkohole aus Cetylalkohol, Stearylalkohol und Mischungen davon wie Cetylstearylalkohol ausgewählt ist bzw. sind.

6. Zusammensetzung nach einem der Ansprüche 1 bis 5, umfassend eine Mischung von C₁₆- und C₁₈-Fettalkoholen, vorzugsweise in einem C₁₆/C₁₈-Massenverhältnis im Bereich von 20/80 bis 80/20 und vorteilhafterweise in einem C₁₆/C₁₈-Massenverhältnis gleich 50/50.

7. Zusammensetzung nach einem der Ansprüche 1 bis 6, wobei die Menge an Fettalkohol zwischen 2,5 Gew.-% und 4,5 Gew.-% und noch weiter bevorzugt zwischen 2,7 Gew.-% und 4 Gew.-%, bezogen auf das Gesamtgewicht der Zusammensetzung, liegt.

8. Zusammensetzung nach einem der Ansprüche 1 bis 7, wobei das anionische Tensid bzw. die anionischen Tenside mit mindestens einer kohlenwasserstoffbasierten C16-C22-Kette aus Tensiden mit mindestens einer Sulfonatfunktion, Acylglutamaten und Mischungen davon ausgewählt ist bzw. sind.

9. Zusammensetzung nach einem der Ansprüche 1 bis 8, wobei das anionische Tensid bzw. die anionischen Tenside mit mindestens einer kohlenwasserstoffbasierten C16-C22-Kette aus den Tensiden der allgemeinen Formel (I) ausgewählt ist bzw. sind: RCOY(CH₂)ₙSO₃M, wobei R für eine gesättigte, lineare oder verzweigte C16-22-Alkylgruppe steht; Y für -O- oder -NR₁- steht, wobei R₁ für eine lineare oder verzweigte C1-C3-Alkylgruppe steht; M aus der Gruppe ausgewählt ist, die durch Wasserstoff, Alkalimetalle, Erdalkalimetalle, die Ammoniumgruppe und organische Amine gebildet wird; n eine ganze Zahl im Bereich von 1 bis 3 ist.

10. Zusammensetzung nach einem der Ansprüche 1 bis 9, wobei es sich bei dem anionischen Tensid mit mindestens einer kohlenwasserstoffbasierten C16-C22-Kette um N-Stearoyl-N-methyltaurat ist, wobei M Na entspricht.

11. Zusammensetzung nach einem der Ansprüche 1 bis 10, wobei das Tensid bzw. die Tenside mit mindestens einer kohlenwasserstoffbasierten C16-C22-Kette in einer Menge zwischen 0,05 Gew.-% und 5 Gew.-%, vorzugsweise zwischen 0,1 Gew.-% und 2 Gew.-% und noch weiter bevorzugt zwischen 0,1 Gew.-% und 1,5 Gew.-%, bezogen auf das Gesamtgewicht der Zusammensetzung, vorliegt bzw. vorliegen.

12. Zusammensetzung nach einem der Ansprüche 1 bis 11, wobei das Massenverhältnis von Fettalkohol (a) zu anionischen Tensiden (b) zwischen 19/2 und 4/1 und noch weiter bevorzugt zwischen 9/1 und 4/1 liegt.

13. Zusammensetzung nach einem der Ansprüche 1 bis 12, wobei das Polyol bzw. die Polyole eine Zahl von Kohlenstoffatomen im Bereich von 2 bis 20 und vorzugsweise von 2 bis 10 umfasst bzw. umfassen.

14. Zusammensetzung nach einem der Ansprüche 1 bis 13, wobei das Polyol bzw. die Polyole 2 bis 12 Hydroxylfunktionen, vorzugsweise 2 bis 8 Hydroxylfunktionen, umfassen.

15. Zusammensetzung nach einem der Ansprüche 1 bis 14, wobei die Polyole aus Ethylenglykol, Propylenglykol, 1,3-Propandiol, Isoprenglykol, Butylenglykol, Dipropylenglykol, Polypropylenglykol, Glycerin, Diglycerin, Erythritol, Pentaerythritol, Arabitol, Adonitol, Sorbitol, Dulcitol, Maltitol, Panthenol, vorzugsweise Glycerin, Propylenglykol, Dipropylenglykol, Butylenglykol und 1,3-Propandiol und noch weiter bevorzugt Glycerin ausgewählt sind.

16. Zusammensetzung nach einem der Ansprüche 1 bis 15, wobei das Polyol bzw. die Polyole in einer Menge zwischen 20 und 60 Gew.-%, vorzugsweise zwischen 20 und 50 Gew.-%, noch besser zwischen 20 und 40 Gew.-% und sogar noch besser zwischen 25 und 35 Gew.-%, bezogen auf das Gesamtgewicht der Zusammensetzung, vorliegt bzw. vorliegen.

17. Zusammensetzung nach einem der Ansprüche 1 bis 16, wobei das Silikonöl bzw. die Silikonöle aus flüchtigen Silikonölen wie Cyclopolydimethylsiloxanen (INCI-Name: Cyclomethicon), wie Cyclopentasiloxan, Cyclohexasiloxan, Octamethylcyclotetrasiloxan, Decamethylcyclopentasiloxan, Dodecamethylcyclohexasiloxan; linearen Silikonen wie Heptamethylhexyltrisiloxan, Heptamethyloctyltrisiloxan, Hexamethyldisiloxan, Octamethyltrisiloxan, Decamethyltetrasiloxan oder Dodecamethylpentasiloxan und nichtflüchtigen Silikonölen wie Polymethylsiloxanen (PDMS) und Phenylpolymethylsiloxanen wie Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilikaten und Polymethylphenylsiloxanen; mit Fettsäuren, Fettalkoholen oder Polyoxyalkylenen modifizierten Polysiloxanen und Mischungen davon ausgewählt ist bzw. sind.

18. Zusammensetzung nach einem der Ansprüche 1 bis 17, umfassend mindestens ein nichtflüchtiges Silikonöl, vorzugsweise ein Silikonöl, das aus Polymethylsiloxanen ausgewählt ist.

19. Zusammensetzung nach einem der Ansprüche 1 bis 18, wobei das Silikonöl bzw. die Silikonöle in einer Menge zwischen 0,06 und 50 Gew.-%, vorzugsweise zwischen 0,6 und 30 Gew.-%, noch weiter bevorzugt zwischen 1,8 und 20 Gew.-% und sogar noch besser zwischen 2,4 und 15 Gew.-%, bezogen auf das Gesamtgewicht der Zusammensetzung, vorliegt bzw. vorliegen.

20. Kosmetisches Verfahren zur Behandlung eines Keratinmaterials, bei dem eine Zusammensetzung gemäß einem der Ansprüche 1 bis 19 auf das Keratinmaterial aufgebracht wird.

21. Verwendung einer Zusammensetzung gemäß einem der Ansprüche 1 bis 19 auf dem Gebiet der Kosmetik und insbesondere zum Pflegen, Schützen und/oder Schminken von Körper- oder Gesichtshaut oder zur Haarpflege.

## Revendications

1. Composition, de préférence composition cosmétique, sous la forme d'une émulsion huile-dans-eau, dans laquelle une phase huileuse est dispersée dans une phase aqueuse, comprenant :
(a) de 2,5 % à 7 % en poids par rapport au poids total de la composition d'au moins un alcool gras saturé en C16-C18 ;
(b) au moins un tensioactif anionique comprenant une chaîne à base d'hydrocarbure en C16-C22, de préférence en C16-C18 ;
(c) au moins un polyol ;
(d) au moins 60 % en poids par rapport au poids total de la phase huileuse d'au moins une huile siliconée ;
le rapport massique alcools gras (a)/tensioactifs anioniques (b) étant compris entre 10:1 et 4:1, et dans laquelle le ou les polyol(s) sont présents en une quantité comprise entre 20 % et 80 % en poids, par rapport au poids total de la composition.

2. Composition selon la revendication 1, dans laquelle le ou les tensioactifs anioniques contiennent seulement une chaîne à base d'hydrocarbure en C16-C22.

3. Composition selon la revendication 2, dans laquelle la différence entre le nombre d'atomes de carbone de la chaîne à base d'hydrocarbure du ou des tensioactifs anioniques (b) et le nombre d'atomes de carbone dans le ou les alcools gras (a) ne doit pas différer de plus de 5 atomes de carbone, et est préférentiellement inférieure ou égale à 4.

4. Composition selon l'une quelconque des revendications 1 à 3, dans laquelle le ou les alcools gras saturés sont choisis parmi l'alcool cétylique ou hexadécanol (C₁₆), l'alcool stéarylique ou octadécanol (C₁₈) qui sont solides à température ambiante et portent avantageusement un groupe -OH en extrémité de chaîne.

5. Composition selon l'une quelconque des revendications 1 à 4, dans laquelle le ou les alcools gras saturés sont choisis parmi l'alcool cétylique, l'alcool stéarylique et leurs mélanges tels que l'alcool cétylstéarylique.

6. Composition selon l'une quelconque des revendications 1 à 5, comprenant un mélange d'alcools gras en C₁₆ et en C₁₈, de préférence en un rapport massique C₁₆/C₁₈ allant de 20/80 à 80/20, et avantageusement en un rapport massique C₁₆/C₁₈ égal à 50/50.

7. Composition selon l'une quelconque des revendications 1 à 6, dans laquelle la quantité d'alcool gras est comprise entre 2,5 % et 4,5 % en poids, et encore plus préférentiellement entre 2,7 % et 4 % en poids par rapport au poids total de la composition.

8. Composition selon l'une quelconque des revendications 1 à 7, dans laquelle le ou les tensioactifs anioniques comprenant au moins une chaîne à base d'hydrocarbure en C16-C22 sont choisis parmi des tensioactifs comprenant au moins une fonction sulfonate, des acylglutamates et leurs mélanges.

9. Composition selon l'une quelconque des revendications 1 à 8, dans laquelle le ou les tensioactifs anioniques comprenant au moins une chaîne à base d'hydrocarbure en C16-C22 sont choisis parmi les tensioactifs de formule générale (I) : RCOY(CH₂)ₙSO₃M dans laquelle R représente un groupe alkyle saturé linéaire ou ramifié en C16-22 ; Y représente -O- ou -NR₁-, R₁ représentant un groupe alkyle linéaire ou ramifié en C1-C3 ; M est choisi dans le groupe formé par hydrogène, les métaux alcalins, les métaux alcalino-terreux, le groupe ammonium et des amines organiques ; n est un entier allant de 1 à 3.

10. Composition selon l'une quelconque des revendications 1 à 9, dans laquelle le tensioactif anionique comprenant au moins une chaîne à base d'hydrocarbure en C16-C22 est N-stéaroyl-N-méthyltaurate, M correspondant à Na.

11. Composition selon l'une quelconque des revendications 1 à 10, dans laquelle le ou les tensioactifs comprenant au moins une chaîne à base d'hydrocarbure en C16-C22 sont présents en quantité comprise entre 0,05 et 5 % en poids, de préférence entre 0,1 et 2 % en poids, et encore plus préférentiellement entre 0,1 % et 1,5 % en poids par rapport au poids total de la composition.

12. Composition selon l'une quelconque des revendications 1 à 11, dans laquelle le rapport massique alcool gras (a)/tensioactifs anioniques (b) est compris entre 19/2 et 4/1 et encore plus préférentiellement entre 9/1 et 4/1.

13. Composition selon l'une quelconque des revendications 1 à 12, dans laquelle le ou les polyols comprennent un nombre d'atomes de carbone allant de 2 à 20, de préférence de 2 à 10.

14. Composition selon l'une quelconque des revendications 1 à 13, dans laquelle le ou les polyols comprennent de 2 à 12 fonctions hydroxyle, de préférence de 2 à 8 fonctions hydroxyle.

15. Composition selon l'une quelconque des revendications 1 à 14, dans laquelle les polyols sont choisis parmi l'éthylène glycol, le propylène glycol, le 1,3-propanediol, l'isoprène glycol, le butylène glycol, le dipropylène glycol, le polypropylène glycol, le glycérol, le diglycérol, l'érythritol, le pentaérythritol, l'arabitol, l'adonitol, le sorbitol, le dulcitol, le maltitol, le panthénol, de préférence le glycérol, le propylène glycol, le dipropylène glycol, le butylène glycol et le 1,3-propanediol, et encore plus préférentiellement le glycérol.

16. Composition selon l'une quelconque des revendications 1 à 15, dans laquelle le ou les polyols sont présents en une quantité comprise entre 20 % et 60 % en poids, préférentiellement entre 20 % et 50 % en poids, mieux encore entre 20 % et 40 % en poids, et encore mieux entre 25 % et 35 % en poids par rapport au poids total de la composition.

17. Composition selon l'une quelconque des revendications 1 à 16, dans laquelle la ou les huiles siliconées sont choisies parmi les huiles siliconées volatiles telles que les cyclopolydiméthylsiloxanes (nom INCI : cyclomethicone), tels que le cyclopentasiloxane, le cyclohexasiloxane, l'octaméthylcyclotétrasiloxane, le décaméthylcyclopentasiloxane, le dodécaméthylcyclohexasiloxane ; les silicones linéaires telles que l'heptaméthylhexyltrisiloxane, l'heptaméthyloctyltrisiloxane, l'hexaméthyldisiloxane, l'octaméthyltrisiloxane, le décaméthyltétrasiloxane ou le dodécaméthylpentasiloxane ; et les huiles siliconées non volatiles telles que les polyméthylsiloxanes (PDMS) et les phényl polyméthylsiloxanes tels que les phényltriméthicones, les phényldiméthicones, les phényltriméthylsiloxydiphénylsiloxanes, les diphényldiméthicones, les diphénylméthyldiphényltrisiloxanes, les 2-phényléthyltriméthylsiloxysilicates et les polyméthylphénylsiloxanes ; les polysiloxanes modifiés par des acides gras, des alcools gras ou des polyoxyalkylènes et leurs mélanges.

18. Composition selon l'une quelconque des revendications 1 à 17, comprenant au moins une huile siliconée non volatile, de préférence une huile siliconée choisie parmi les polyméthylsiloxanes.

19. Composition selon l'une quelconque des revendications 1 à 18, dans laquelle la ou les huiles siliconées sont présentes en une quantité comprise entre 0,06 % et 50 % en poids, de préférence entre 0,6 % et 30 % en poids, encore plus préférentiellement entre 1,8 % et 20 % en poids, et mieux encore entre 2,4 % et 15 % en poids par rapport au poids total de la composition.

20. Procédé de traitement cosmétique d'une matière kératinique, dans lequel une composition telle que définie dans l'une quelconque des revendications 1 à 19 est appliquée sur la matière kératinique.

21. Utilisation d'une composition telle que définie dans l'une quelconque des revendications 1 à 19, dans le domaine des produits cosmétiques, et en particulier pour le soin, la protection et/ou le maquillage de la peau du corps ou du visage, ou pour le soin des cheveux.
